# EUROPÄISCHE PATENTSCHRIFT

(11) **EP 0 814 088 B1**
(45) Veröffentlichungstag und Bekanntmachung des Hinweises auf die Patenterteilung: **11.05.2005**
(21) Anmeldenummer: 97109453.7
(22) Anmeldetag: 11.06.1997
(51) Int. Cl.: C07H 15/04, C11D 1/66, C11D 1/04

(54) **Acylierte Carboxyalkylsaccharide, Verfahren zu deren Herstellung und deren Verwendung in Waschmitteln**
Acylated carboxyalkyl saccharides, method for their preparation and their use in detergents
Saccharides carboxylalkyl acylés, procédé de préparation et leur utilisation dans les détergents

(30) Priorität: 19.06.1996 DE 19624345
(43) Veröffentlichungstag der Anmeldung: 29.12.1997
(73) Patentinhaber: SÜDZUCKER AKTIENGESELLSCHAFT MANNHEIM/OCHSENFURT, 68165 Mannheim (DE)
(72) Erfinder: Ehrhardt, Sonja, Dr., 64521 Gross-Gerau (DE); Begli, Alireza Haji, Dr., 67305 Ramsen (DE); Kunz, Markwart, Dr., 67550 Worms (DE)
(74) Vertreter: Schrell, Andreas, Dr.

(56) Entgegenhaltungen:
- US-A- 3 634 392
- B. DEAN ET AL. : "Synthesis of multivalent beta-lactosyl cluster as potential tumor metastasis inhibitors" CARBOHYDRATE RESEARCH, Nr. 245, 1993, Seiten 175-192, XP002073665
- W. BINDER AND W. SCHMID: " Synthesis of a symmetric multivalent molecule containing four carbohydrate substituents" MONATSHEFTE FÜR CHEMIE, Bd. 126, - 1995 Seiten 923-931, XP002073666
- BEYER H.: 'Lehrbuch der organischen Chemie', 1978, HIRZEL VERLAG STUTTGART
- FALBE J.: 'Römpp Chemie Lexikon', 1989, GEORG THIEME VERLAG

## Beschreibung

Die Erfindung betrifft acylierte Carboxyalkylsaccharide, Verfahren zu deren Herstellung sowie deren Verwendung in Waschmitteln als Komplexierungsmittel und Bleichmittelaktivator gemäß der Patentansprüche.

Waschmittel enthalten im allgemeinen Tenside, Bleichmittel, als sogenannte Builder bezeichnete Gerüststoffe sowie optische Aufheller und Hilfsstoffe. Die Bleichmittel dienen dem Entfärben der zu waschenden Materialien, in dem die unerwünscht färbenden Begleitstoffe durch oxidierende oder reduzierende Chemikalien zerstört werden. Als Bleichmittel wird häufig Natriumperborat verwendet, welches oberhalb von 60°C zunehmend Perhydroxyl-Anionen als Aktivsauerstoff abspaltet und so eine Reihe von Verschmutzungen abbaut. Bei geringeren Temperaturen ist die Bleichwirkung jedoch unzureichend, so daß sogenannte Bleichmittelaktivatoren, wie Tetraacetylethylendiamin eingesetzt werden. Die Aktivatoren werden durch die Bleichmittel perhydrolysiert und setzen dabei bleichaktive Spezies, wie die Persäuren, frei.

Die Builder dienen als Komplexierungsmittel und eliminieren vorwiegend Calcium- und Magnesiumionen.

Heutzutage wird die Auswahl der als Bleichmittelaktivator und Builder einsetzbaren Waschmittelbestandteile in erheblichem Maße von den Gesichtspunkten des Umweltschutzes und der Energieeinsparung beeinflußt. Daher werden beispielsweise Aktivatoren auf der Basis nachwachsender Rohstoffe, wie Kohlenhydrate, verwendet, die günstigere Nutzungsgrade und eine bessere biologische Verträglichkeit aufweisen. Ebenso sind aus DE 2 149 737 und US-Patent 3,634,392 carboxymethylierte Oligosaccharide bekannt, die als Builder in Waschmitteln verwendet werden.

Die auf der Basis von Kohlenhydraten entwickelten Verbindungen werden jedoch aufgrund ihrer Struktur entweder nur als Komplexierungsmittel beziehungsweise Builder oder nur als Bleichmittelaktivator in Waschmitteln verwendet. Die Waschmittel müssen also für jede der beiden erforderlichen Funktionen eine eigene Substanz enthalten. Aus Dean et al. (Carbohydrate Research 245 (1993), 175-192) sind bestimmte acetylierte Lactosederivate als Inhibitoren von Tumorwachstum bekannt. Aus Binder und Schmid (Monatshefte für Chemie 126 (1995), 923-931) sind bestimmte acetylierte Glucosederivate bekannt. Die dort offenbarten genannten Derivate werden aus der vorliegenden Lehre ausgeklammert.

Das der vorliegenden Erfindung zugrundeliegende technische Problem liegt also darin, Verbindungen bereitzustellen, die gleichzeitig als (Co-)Builder und Bleichmittelaktivator wirken sowie eine besonders gute Umweltverträglichkeit und hohe Wirkungseffizienz aufweisen.

Die Lösung dieses technischen Problems liegt in der Bereitstellung von acylierten Kohlenhydraten mit mindestens einer mit dem Kohlenhydrat veretherten Carboxyalkyl-Gruppe der folgenden allgemeinen Formel

KH[-O-CHR₁-(CH₂)ₚ - (COO⁻oder COOH)]ₙ [-O-CO-R₂]ₘ,

in der KH (Kohlenhydrat) ein Monosaccharid, Disaccharid, Trisaccharid, Polysaccharid wie Polyglucan, insbesondere Cellulose, Stärke oder Maltodextrine oder Polyfructan, insbesondere Inulin, ist und,
wenn KH ein Monosaccharid ist, n = 1 bis 4 und m = 1 bis 4 mit n + m = 2 bis 5;
wenn KH ein Disaccharid ist, n = 1 bis 7 und m = 1 bis 7 mit n + m = 2 bis 8;
wenn KH ein Trisaccharid ist, n = 1 bis 10 und m = 1 bis 10 mit n + m = 2 bis 11;
wenn KH ein Polysaccharid, wie Polyfructan oder Polyglucan ist, n = 0,2 bis 2,8 und m = 0,2 bis 2,8 (bezogen auf eine Monosaccharideinheit) mit n + m = 0,4 bis 3 ist, wobei
R₁ = H oder ein 1 - 9 Kohlenstoffatome aufweisender Rest, insbesondere Alkyl- oder Acylrest ist und
R₂ ein 1 - 9 Kohlenstoffatome aufweisender Rest, insbesondere Alkyl- oder Acylrest ist und wobei p=0-9 ist, mit Ausnahme von 2,3,6,2',3',.4',6'-Heptaacetyl-1-carboxymethyl-β-lactose, 2,3,6,2',3', 4',6'-Heptaacetyl-1-carboxypropyl-β-lactose, 2,3, 4,6-Tetra-O-acetyl-1-carboxymethyl-β-D-glucopyranosid und 2,3,4,6-Tetra-O-acetyl-1-carboxypropyl-β-D-glucopyranosid.

Die erfindungsgemäßen Verbindungen stellen acylierte Kohlenhydrate mit mindestens einer Carboxyalkyl-Gruppe dar, wobei durch die in einer Verbindung realisierte Kombination von Carboxyalkyl-Gruppe und Acyl-Gruppe Verbindungen erhalten werden, die gleichzeitig als Aktivator und (Co-) Builder eine hohe Effektivität, gute Wasserlöslichkeit und ausgezeichnete Umweltverträglichkeit aufweisen. Die mindestens eine Carboxyalkylgruppe [-O-CHR₁-(CH₂)ₚ-(COO⁻ oder COOH)] liegt in Säure (COOH) oder in Salzform (COO⁻) vor und ist über ein Sauerstoffatom mit dem Kohlenhydrat verethert. Die mindestens eine Carboxygruppe [-O-CO-R₂] ist mit dem Kohlenhydrat verestert.

Die Erfindung betrifft also acylierte Kohlenhydrate mit mindestens einer Carboxyalkyl-Gruppe, wobei die Kohlenhydrate aus der Gruppe der Monosaccharide, Disaccharide, Trisaccharide oder Polysaccharide wie Polyfructane, zum Beispiel Inulin oder Polyglucane zum Beispiel Cellulose, Stärke oder Maltodextrine ausgewählt sind sowie deren Verwendung in Waschmitteln als (Co-)Builder mit gleichzeitiger Bleichmittel-aktivierender Wirkung.

In besonders bevorzugter Weise werden als Kohlenhydrate Glucose, Fructose, Saccharose, Palatinose, Maltose, Lactose, Raffinose, Trehalulose, Polyfructane, Inulin, Polyglucane, Cellulose, Stärke, Maltodextrine oder eine Mischung davon verwendet.

Im Zusammenhang mit der vorliegenden Erfindung werden unter Polysacchariden, also insbesondere Polyfructanen, wie Inulin oder Polyglucanen, wie Cellulose oder Stärke, auch abgebaute Polysaccharide, also abgebaute Polyfructane oder Polyglucane verstanden. Abgebaute Polyfructane oder Polyglucane entstehen durch enzymatische oder säure-katalysierte Spaltung der Polyglucane oder Polyfructane und zeichnen sich zum Beispiel durch eine geringere Viskosität und verringerte Kettenlängen aus. Der Abbau der Polyglucane oder Polyfructane wird bis zu einer Viskosität und/oder Kettenlänge des Rohstoffes durchgeführt, die für das zu synthetisierende Derivat erwünscht ist. Die abgebauten Polyfructane oder Polyglucane werden erfindungsgemäß als Ausgangsstoff für die Derivatisierung gemäß der Erfindung bevorzugt.

Die Anzahl der Carboxyalkyl-Gruppen pro Kohlenhydrat, das heißt der Veretherungsgrad DS, beträgt bei Monosacchariden 1 bis 4, bei Disacchariden 1 bis 7 und bei Trisacchariden 1 bis 10. Der Veretherungsgrad DS beträgt bei Polysacchariden 0,2 bis 2,8, hier bezogen auf eine Monosaccharideinheit. In besonders bevorzugter Weise werden Veretherungsgrade von 1 bis 3 bei Di- und Trisacchariden, von 0,2 bis 1,0 bei Polysacchariden sowie um 1 bei Monosacchariden bevorzugt. Die Carboxyalkyl-Gruppe weist bevorzugt 1 bis 11 Kohlenstoffatome auf und ist besonders bevorzugt eine Carboxymethyl- oder Carboxyethylgruppe.

Die Anzahl der Acylgruppen pro Kohlenhydrat, das heißt der Acylierungsgrad, beträgt bei Monosacchariden 1 bis 4, bei Disacchariden 1 bis 7, bei Trisacchariden 1 bis 10. Der Acylierungsgrad beträgt bei Polysacchariden 0,2 bis 2,8, hier bezogen auf eine Monosaccharideinheit. In besonders bevorzugter Weise werden die mindestens eine Carboxyalkyl-Gruppen aufweisenden Kohlenhydrate vollständig acyliert, das heißt, alle nicht veretherten Hydroxygruppen des Kohlenhydrats werden acyliert. In unerwarteter und vorteilhafter Weise werden die Carboxyalkyl-Gruppen dabei nicht verändert. Die Acylgruppen weisen in bevorzugter Weise 2 bis 10 Kohlenstoffatome, in besonders bevorzugter Weise 2 bis 6 Kohlenstoffatome auf. Insbesondere sieht die Erfindung die Acetylierung der carboxyalkylierten Kohlenhydrate vor.

Carboxyalkylierte Kohlenhydrate können zur Herstellung der erfindungsgemäßen Verbindungen durch Veretherung von α-Halogencarbonsäuren mit Kohlenhydraten hergestellt werden (US 3 634 392, DE 2 149 737, van Bekkum et al., Carbohydr. Res. 271 (1995), 101). Die carboxyalkylierten Kohlenhydrate können auch hergestellt werden, indem eine Michael-analoge Addition von Kohlenhydraten an beispielsweise Acrylnitril mit anschließender Verseifung der Nitrilgruppe zur Carboxygruppe durchgeführt wird (US 3,068,220; US 3,161,359; AT 369 383). Die Herstellung von CMC (Carboxymethylcellulose) ist beschrieben in C.V. Nikonovich et al. J. Polym. Sci. Symposium Nr. 42, 1625 (1973). Die derartig hergestellten carboxyalkylierten Kohlenhydrate können auf verschiedene Weise acyliert, insbesondere vollständig acyliert werden. Als Acylierungsmittel lassen sich Carbonsäureanhydride oder Carbonsäurechloride in Gegenwart verschiedener Katalysatoren verwenden. Überraschenderweise bleibt die mindestens eine Carboxyalkyl-Gruppe bei der Acylierung erhalten, so daß die acylierten, insbesondere vollständig acylierten Kohlenhydrate bei gleichzeitig vorhandener, für den Waschvorgang ausreichender Wasserlöslichkeit durch die Acylgruppen in vorteilhafter Weise als Bleichmittelaktivator und Builder eingesetzt werden können.

Als α-Halogencarbonsäuren zur Veretherung kommen insbesondere die Chlor- und Bromcarbonsäuren in Betracht. Carboxymethylierungen erfolgen in stark alkalischer, wässriger Lösung bei Temperaturen von 20°C bis 80°C, bevorzugt von 20°C bis 50°C. Als Basen werden anorganische Basen, wie Natriumhydroxid oder Kaliumhydroxid eingesetzt. Carboxyethylierungen können durch Addition von Kohlenhydraten an Acrylnitril und anschließende Verseifung des Nitrils zur Säure durchgeführt werden. Die Additionsreaktion wird in stark alkalischer Lösung bei Temperaturen von 20°C bis 80°C, bevorzugt von 30°C bis 50°C, durchgeführt. Die erforderliche Alkalität wird durch Zugabe von Alkalihydroxiden, wie Natrium- oder Kaliumhydroxid, erreicht. Die Hydrolyse der Nitrilgruppen zur Carboxygruppe erfolgt bevorzugt zweistufig, und zwar wird zunächst durch Hydrolyse mit 30% Wasserstoffperoxid in schwach basischer Lösung bei pH-Werten von 8 bis 9 das Amid gebildet, wobei die Reaktionstemperatur 50°C bis 60°C und die Reaktionszeit 2 bis 4 Stunden beträgt. Die notwendige Alkalität der Lösung wird durch Zugabe von Kalium- oder Natriumhydroxid erreicht. In einer zweiten Stufe wird das Amid bei höheren pH-Werten von 12 bis 14 zur Carbonsäure verseift. Auch hier wird die notwendige Alkalität der Lösung durch Zugabe von Natrium- oder Kaliumhydroxid erreicht. Die Reaktionstemperatur beträgt 20°C bis 50°C, bevorzugt 20°C bis 30°C. Die Reaktionszeit liegt bei maximal 16 Stunden. Der Veretherungsgrad DS wird in beiden Verfahrensweisen durch das Mol-Verhältnis Kohlenhydrat (KH)-OH/Reagenz eingestellt und muß für Trisaccharide bei 1 bis 10, für Disaccharide bei 1 bis 7 und für Monosaccharide bei 1 bis 4 liegen. Bei der Verwendung von Polysacchariden als Kohlenhydrat liegt der Veretherungsgrad DS bei 0,2 bis 2,8, bezogen auf eine Monosaccharideinheit. Der Veretherungsgrad kann entweder durch NMR-spektroskopische oder titrimetrische Methoden bestimmt werden.

Die derartig hergestellten carboxyalkylierten Kohlenhydrate, insbesondere die Mono-, Di- und Trisaccharidderivate, werden mit Carbonsäureanhydriden von Carbonsäuren mit 2 bis 10, vorzugsweise 2 bis 6, Kohlenstoffatomen in Gegenwart organischer Basen wie Pyridin, 4-Dimethylaminopyridin oder anorganischer Basen, wie Natriumacetat oder in Gegenwart von Lewis-Säuren, wie Zinkchlorid, acyliert. Die Acylierung von Polysacchariden wie, vorzugsweise abgebauter, Cellulose oder Stärke mit den genannten Carbosäureanhydriden wird bevorzugt in Gegenwart von Brönstedt-Säuren wie Schwefelsäure oder Eisessig durchgeführt. Inulinderivate können mit Hilfe von Brönstedt-Säuren oder basischen Katalysatoren acyliert werden. Die Reaktionstemperatur wird in einem Bereich von 0°C bis 70°C eingestellt, und die Reaktionszeit beträgt 3 bis 72 Stunden. Die Acylierung kann auch mit Carbonsäurechloriden von Carbonsäuren mit 2 bis 10, vorzugsweise 2 bis 6, Kohlenstoffatomen in Gegenwart organischer Basen, wie Pyridin oder 4-Dimethylaminopyridin bei Temperaturen von 0°C bis 50°C erfolgen, wobei Temperaturen von 0°C bis 20°C bevorzugt werden. Der Acylierungsgrad wird durch das Verhältnis carboxyalkyliertes Kohlenhydrat-OH/Reagenz eingestellt, wobei eine partielle oder vollständige Acylierung der noch vorhandenen OH-Gruppen vorgenommen werden kann.

Die Identifizierung der erfindungsgemäßen Verbindungen erfolgt spektroskopisch: Die Esterschwingungen können um 1750, 1250 und 1080 cm⁻¹ und die der Carboxyalkyl-Gruppe um 1710 cm⁻¹ beobachtet werden (IR-Spektroskopie). Im Protonen-NMR erscheinen die Signale der CH-CO-O-Gruppe um 2 ppm. Die CO-Signale beider Substituenten erscheinen im ¹³C-NMR im Bereich 185 bis 170 ppm.

Die acylierten carboxyalkylierten Kohlenhydrate der vorliegenden Erfindung eignen sich besonders zur Verwendung in Waschmittelformulierungen als Bleichmittelaktivatoren mit Builder-Eigenschaften.

Die folgenden Ausführungsbeispiele erläutern die Erfindung.

### Beispiel 1 Vollständige Acetylierung von carboxymethylierter Saccharose (DS 2) mit Zinkchlorid als Katalysator

In 24 ml Acetanhydrid wird 1,03 g getrocknetes Zinkchlorid suspendiert; das Gemisch wird auf 0°C abgekühlt und mit 10 g carboxymethylierter Saccharose (DS 2) versetzt. Die Reaktionsmischung wird langsam auf Raumtemperatur erwärmt und nach 60 h unter Zugabe von 30 ml Eiswasser hydrolysiert. Nach der Extraktion mit Dichlormethan und Trocknen der organischen Phase läßt sich vollständig acetylierte, carboxymethylierte Saccharose (DS 2) in 12,6 g (80% Ausbeute) isolieren.

### Beispiel 2 Vollständige Acetylierung von carboxyethylierter Saccharose (DS 2) in Gegenwart von Zinkchlorid

In 24 ml Acetanhydrid wird 1,03 g getrocknetes Zinkchlorid suspendiert; das Gemisch wird bei 0°C mit 10 g carboxyethylierter Saccharose (DS 2) versetzt, langsam auf 30°C erwärmt und nach 72 h mit 30 ml Eiswasser hydrolysiert. Nach Extraktion mit Dichlormethan und Trocknung der organischen Phase wird vollständig acetylierte, carboxymethylierte Saccharose (DS 2) als farbloser Feststoff in 84% Ausbeute (12,7 g) gewonnen.

### Beispiel 3 Vollständige Acetylierung von carboxyethylierter Glucose (DS 1) in Gegenwart von Natriumacetat

Das Reaktionsgemisch aus 0,2 g Natriumacetat, 24 ml Acetanhydrid und 2,5 g carboxyethylierter Glucose (DS 1) wird 40 h bei 60°C erhitzt. Man läßt die Reaktionslösung abkühlen und entfernt unter Vakuum das Lösungsmittel. Der Rückstand wird in Dichlormethan dispergiert, die organische Phase wird mit Aktivkohle gereinigt und das Lösungsmittel abdestilliert. Die vollständig acetylierte, carboxyethylierte Glucose (DS 1) wird in 75% Ausbeute (3,1 g) erhalten.

### Beispiel 4 Partielle Acetylierung von carboxymethylierter Maltose (DS 2) mit Acetylchlorid in Pyridin

Zu einer Lösung von 5 g carboxymethylierter Maltose in 30 ml Pyridin werden bei 0°C 7 ml Acetylchlorid zugetropft, und nach 2 h bei dieser Temperatur wird die Reaktionsmischung auf 40 ml Eiswasser gegossen. Die erhaltene Lösung wird mehrmals mit Dichlormethan extrahiert, die organische Phase mit Aktivkohle aufgereinigt, getrocknet und eingeengt. Es wird teilweise acetylierte, carboxymethylierte Maltose (DS 2) (6,5 g) isoliert.

### Beispiel 5 Partielle Acetylierung von carboxyethylierter Lactose (DS 2) mit Acetylchlorid in Pyridin

Zu einer Lösung von 5 g carboxyethylierter Lactose in 30 ml Pyridin werden bei 0°C 7 ml Acetylchlorid zugetropft, und nach 2 h bei dieser Temperatur wird die Reaktionsmischung auf 40 ml Eiswasser gegossen. Die erhaltene Lösung wird mehrmals mit Dichlormethan extrahiert, die organische Phase mit Aktivkohle aufgereinigt, getrocknet und eingeengt. Es wird teilweise acetylierte, carboxyethylierte Lactose (DS 2) (5,6 g) erhalten.

### Beispiel 6 Vollständige Acylierung von carboxyethylierter Saccharose (DS 2) mit Capronsäurechlorid in Pyridin

Zu einer Lösung von 2 g carboxyethylierter Saccharose (DS 2) in 30 mL Pyridin werden bei 0°C 8.7 mL Capronsäurechlorid zugetropft, und nach 2 h bei dieser Temperatur wird die Reaktionsmischung auf 30 mL Eiswasser gegossen. Die erhaltene Lösung wird mehrmals mit Dichlormethan extrahiert, die organische Phase mit Aktivkohle (Norit SK) aufgereinigt und eingeengt. Die vollständig acylierte, carboxyethylierte Saccharose (DS 2) wird in 70% (3 g) Ausbeute erhalten.

### Beispiel 7 Partielle Acetylierung von Carboxymethylinulin Na-Salz (CMI, DS 1) mit Acetanhydrid/Eisessig.

Zu einer Suspension von 10 g (0,041 mol) CMI in 50 ml Eisessig, versetzt mit 0,3 ml konzentrierter Schwefelsäure werden unter Rühren bei Raumtemperatur ein Gemisch von 10 ml Eisessig in 25 ml Acetanhydrid zugetropft und anschließend bei 50°C drei Stunden erwärmt.

Das Produkt wird durch Filtration und anschließendes Neutralwaschen mit Aceton/Wasser als weißer Feststoff isoliert.

### Beispiel 8 Partielle Acetylierung von Carboxymethylcellulose Na-Salz (CMC, DS 0,65-0,95) mit Acetanhydrid/Eisessig.

Zu einer Suspension von 10 g CMC (Walocel CRT 30 GA, Wolff Walsrode) in 50 ml Eisessig, versetzt mit 0,3 ml konzentrierter Schwefelsäure werden unter Rühren bei Raumtemperatur ein Gemisch von 10 ml Eisessig in 25 ml Acetanhydrid zugetropft und anschließend bei 50°C drei Stunden erwärmt.

Das Produkt wird durch Filtration und anschließendes Neutralwaschen mit Aceton/Wasser als weißer Feststoff isoliert.

### Beispiel 9 Partielle Acetylierung von Carboxymethylstärke Na-Salz (CMSt, DS 1) mit Acetanhydrid/Eisessig.

Zu einer Suspension von 10 g CMS in 50 ml Eisessig, versetzt mit 0,3 ml konzentrierter Schwefelsäure, werden unter Rühren bei Raumtemperatur ein Gemisch von 10 ml Eisessig in 25 ml Acetanhydrid zugetropft und anschließend bei 50°C drei Stunden erwärmt.

Das Produkt wird durch Filtration und anschließendes Neutralwaschen mit Aceton/Wasser als weißer Feststoff isoliert.

### Beispiel 10 Aktivatortest

Zu 1,15 g Natriumperborat in 30 ml 0,1 N Natronlauge werden 0,026 g Ethylendiamintetraessigsäure und der Aktivator (0,08 M) hinzugegeben. Nach 30 min. bei 30°C wird eine 7,5 ml Probe bei 0°C rasch zu 8,75 ml 5% Schwefelsäure getropft. Mit 0,1 N Cer- (IV) - ammoniumsulfatlösung wird die Mischung gegen Ferroin titriert (Bestimmung des Wasserstoffperoxidgehaltes). Anschließend wird die Lösung mit 10 ml 10% Kaliumiodidlösung versetzt und iodometrisch mit 0,1 N Natriumthiosulfatlösung titriert (Bestimmung des Persäuregehaltes).

Die Ergebnisse sind in der Tabelle I dargestellt.

Peracetatbildung (in Mol %) in Gegenwart von Naperborat in 0,1 n NaOH bei 30°C nach 30 Minuten

**Tabelle I**

| Substanz | Mol % | Substanz | Mol % |
|---|---|---|---|
| CMS (1)-Acetat | 20 | CEL (2)-Acetat | 19 |
| CMS (2)-Acetat | 18 | CMM (2)-Acetat | 18 |
| CES (1)-Acetat | 20 | CEM (2) -Acetat | 17 |
| CES (2)-Acetat | 19 | CEG (1)-Acetat | 20 |
| CMI (1)-Acetat | 30 | CMSt(1)-Acetat | 18 |
| CMC (0,65-0,95)-Acetat | 26 | | |

- CMS (1)-Acetat:: vollständig acetylierte Carboxymethylsaccharose mit DS 1
- CMS (2)-Acetat: peracetylierte Carboxymethylsaccharose mit DS 2
- CES (1)-Acetat: vollständig acetylierte Carboxyethylsaccharose mit DS 1
- CES (2)-Acetat: vollständig acetylierte Carboxyethylsaccharose mit DS 2
- CML (2)-Acetat: vollständig acetylierte Carboxymethyllactose mit DS 2
- CMM (2)-Acetat: vollständig acetylierte Carboxymethylmaltose mit DS 2
- CEM (2)-Acetat: vollständig acetylierte Carboxyethylmaltose mit DS 2
- CEG (1)-Acetat: vollständig acetylierte Carboxyethylglucose mit DS 1
- CMI (1)-Acetat: Partiell acetyliertes Carboxymethylinulin mit DS 1
- CMC (0,65-0,95)-Acetat: Partiell acetylierte Carboxymethylcellulose mit DS 0,65-0,95
- CMSt (1)-Acetat: Partiell acetylierte Carboxymethylstärke mit DS 1

Die Mol % beziehen sich bei den Polysacchariden (CMI, CMSt, CMC) auf die Monosaccharideinheit.

Das Beispiel zeigt, daß die erfindungsgemäßen Verbindungen bei 30°C bleichaktive Spezies, die Persäuren, entwickeln.

### Beispiel 11 Komplexierungseigenschaften der vollständig acylierten carboxyalkylierten Verbindungen

Das Komplexierungsvermögen der erfindungsgemäß verwendeten Verbindungen wurde gegenüber Calcium-Ionen nach Hampshire, einem gängigen Testverfahren zur Bestimmung des Komplexierungsverhaltens von Waschmittelkomponenten gegenüber Calcium-Ionen, ermittelt. Die Reaktionslösung des Aktivatortests, 1,72 g vollständig acetylierte carboxymethylierte Saccharose (DS 1), 0.026 g Ethylendiamintetraessigsäure und 1,15 g Natriumperborat in 30 mL 0.1 N Natronlauge wird nach 30 min bei 30°C mit 10 mL, 2 Gew.-% Natriumcarbonatlösung versetzt, mit 1 N Natronlauge auf pH 11 eingestellt und bis 100 mL aufgefüllt. Anschließend wird mit 0.25 M Calciumacetatlösung bis zur Trübung titriert. Die in Tabelle II dargestellten Untersuchungsergebnisse basieren auf einer Aktivatorkonzentration von 0,08 molarer Lösung.

**Tabelle II**

| Substanz | Ca-Carbonatmenge (mg) |
|---|---|
| CMS (1)-Acetat | 86 |
| CMS (2)-Acetat | 98 |
| CES (1)-Acetat | 82 |
| CMG (1)-Acetat | 41 |
| CMI (1)-Acetat | 96 |
| CMC (0,65-0,95)-Acetat | 56 |

Das Beispiel zeigt, daß die bleichmittelaktivierenden Verbindungen gleichzeitig in vorteilhafter Weise als Komplexierungsmittel eingesetzt werden können.

Die folgenden Beispiele 12 und 13 verdeutlichen, daß Kohlenhydrate die nur eine Carboxyalkylgruppe oder nur acyliert sind auch nur entweder die Bleichmittel aktivierende oder nur die komplexierende Eigenschaft aufweisen, die in vorteilhafter Weise in den erfindungsgemäßen Verbindungen gleichzeitig verwirklicht sind.

### Beispiel 12: Aktivator- und Hampshire-Text mit carboxyethylierter Saccharose

1,15 g Natriumperborat in 30 mL 0,1 N Natronlauge werden nacheinander mit 0,026 g Ethylendiamintetraessigsäure und 1,16 g carboxyethylierter Saccharose (DS 2) versetzt. Nach 30 min. bei 30° C wird von einer 3 mL Probe iodometrisch, wie vorstehend beschrieben, der Persäuregehalt ermittelt. Dieser liegt bei 2 Mol-%.

Aus der gleichen Reaktionslösung wird ebenfalls nach 30 min. bei 30° C von einer 20 mL Probe, wie vorstehend beschrieben, das Calciumbindevermögen nach Hampshire ermittelt. Dieses liegt bei 91 mg Calciumcarbonat/1 g Substanz.

### Beispiel 13: Aktivator- und Hampshire-Test mit Saccharoseoctaacetat

1,15 g Natriumperborat in 30 mL 0,1 N Natronlauge werden nacheinander mit 0,026 g Ethylendiamintetraessigsäure und 1,63 g Saccharoseoctaacetat versetzt. Nach 30 min. bei 30° C wird von einer 3 mL Probe iodometrisch, wie vorstehend beschrieben, der Persäuregehalt ermittelt. Dieser liegt bei 12 Mol-%.

Aus der gleichen Reaktionslösung wird ebenfalls nach 30 min bei 30° C von einer 20 mL Probe, wie vorstehend beschrieben, das Calciumbindevermögen nach Hampshire ermittelt. Dieses liegt bei 35 mg Calciumcarbonat/1 g Substanz.

## Patentansprüche

1. Acylierte Kohlenhydrate mit mindestens einer mit dem Kohlenhydrat veretherten Carboxyalkyl-Gruppe der allgemeinen Formel
KH[-O-CHR₁-(CH₂)ₚ-(COOH oder COO⁻)]ₙ[-O-CO-R₂]ₘ
in der KH (Kohlenhydrat) ein Monosaccharid, Disaccharid, Trisaccharid oder Polysaccharid ist und,
wenn KH ein Monosaccharid ist, n = 1 bis 4 und m = 1 bis 4 mit n + m = 2 bis 5 ist,
wenn KH ein Disaccharid ist, n = 1 bis 7 und m = 1 bis 7 mit n + m = 2 bis 8 ist,
wenn KH ein Trisaccharid ist, n = 1 bis 10 und m = 1 bis 10 mit n + m = 2 bis 11 ist und
wenn KH ein Polysaccharid ist, n = 0,2 bis 2,8 und m = 0,2 bis 2,8 mit n + m = 0,4 bis 3 (bei Polysaccharid bezogen auf die Monosaccharideinheit des KH) ist und
wobei R₁ = H oder ein 1 bis 9 Kohlenstoffatome aufweisender Rest ist und
R₂ ein 1 bis 9 Kohlenstoffatome aufweisender Rest und p = 0 bis 9 ist, mit Ausnahme von 2,3,6,2',3',4',6'-Heptaacetyl-1-carboxymethyl-β-lactose, 2,3,6,2',3',4',6'-Heptaacetyl-1-carboxypropyl-β-lactose, 2,3,4,6-Tetra-O-acetyl-1-carboxymethyl-β-D-glucopyranosid und 2,3,4,6-Tetra-O-acetyl-1-carboxypropyl-β-D-glucopyranosid.

2. Verbindung nach Anspruch 1, wobei R₁ ein Alkyl- oder Acylrest ist und/oder R₂ ein Alkyl- oder Acylrest ist.

3. Verbindung nach Anspruch 1 oder 2, **dadurch gekennzeichnet, daß** das Kohlenhydrat (KH) Glucose, Fructose, Saccharose, Maltose, Palatinose, Raffinose, Lactose, Trehalulose, ein Polyfructan oder ein Polyglucan ist.

4. Verbindung nach Anspruch 3, wobei das Polyfructan Inulin ist.

5. Verbindung nach Anspruch 3, wobei das Polyglucan Cellulose, Stärke oder ein Maltodextrin ist.

6. Verbindung nach einem der Ansprüche 1 bis 5, **dadurch gekennzeichnet, dass** wenn das Kohlenhydrat (KH) ein Monosaccharid ist, n = 1 ist, wenn KH ein Di- oder Trisaccharid ist, n = 1 bis 3 ist und wenn KH ein Polysaccharid ist, n = 0,2 bis 1 ist.

7. Verbindung nach einem der Ansprüche 1 bis 6, **dadurch gekennzeichnet, dass** das Kohlenhydrat (KH) vollständig acyliert ist.

8. Verbindung nach Anspruch 7, **dadurch gekennzeichnet, dass** das Kohlenhydrat (KH) vollständig acetyliert ist.

9. Verbindung nach einem der Ansprüche 1 bis 8, **dadurch gekennzeichnet, dass** R₁ gleich H mit p = 0 oder p = 1 ist.

10. Verfahren zur Herstellung acylierter, mindestens eine veretherte Carboxyalkyl-Gruppe aufweisender Kohlenhydrate nach einem der Ansprüche 1 bis 9, wobei carboxyalkylierte Kohlenhydrate mit Carbonsäureanhydriden oder Carbonsäurechloriden in Gegenwart eines Katalysators umgesetzt werden.

11. Verfahren nach Anspruch 10, **dadurch gekennzeichnet, dass** das Kohlenhydrat (KH) ein Mono-, Di-, Trisaccharid oder Polysaccharid ist, die Carboxyalkyl-Gruppe eine Carboxymethyl- oder Carboxyethylgruppe ist und wobei Carbonsäureanhydride oder -chloride von C2 bis C10verwendet werden.

12. Verfahren nach Anspruch 11, **dadurch gekennzeichnet, dass** Carbonsäureanhydride oder -chloride von C2 bis C6 Carbonsäuren verwendet werden.

13. Verfahren nach einem der Ansprüche 10 bis 11, **dadurch gekennzeichnet, dass** der Katalysator Pyridin, 4-Dimethylaminopyridin, Natriumacetat, Zinkchlorid oder eine Brönstedt-Säure ist.

14. Verwendung von acylierten, mindestens eine veretherte Carboxyalkyl-Gruppe aufweisenden Kohlenhydraten nach einem der Ansprüche 1 bis 9 in Waschmitteln als Komplexierungsmittel mit gleichzeitiger bleichmittelaktivierender Wirkung.

## Claims

1. Acylated carbohydrates with at least one carboxyalkyl group etherified with the carbohydrate, with the general formula
KH[-O-CHR₁-(CH₂)ₚ-(COOH or COO⁻)]ₙ[-O-CO-R₂]ₘ
in which KH (carbohydrate) is a monosaccharide, disaccharide, trisaccharide or polysaccharide and,
if KH is a monosaccharide, n = 1 to 4 and m = 1 to 4 with n + m = 2 to 5,
if KH is a disaccharide, n = 1 to 7 and m = 1 to 7 with n + m = 2 to 8,
if KH is a trisaccharide, n = 1 to 10 and m = 1 to 10 with n + m = 2 to 11 and
if KH is a polysaccharide, n = 0.2 to 2.8 and m = 0.2 to 2.8 with n + m = 0.4 to 3 (in the case of polysaccharide based on the monosaccharide unit of the KH) and
in which R₁ = H or a radical having 1 to 9 carbon atoms and
R₂ is a radical having 1 to 9 carbon atoms and p = 0 to 9, with the exception of 2,3,6,2',3',4',6'-heptaacetyl-1-carboxymethyl-β-lactose, 2,3,6,2',3',4',6'-heptaacetyl-1-carboxypropyl-β-lactose, 2,3,4,6-tetra-O-acetyl-1-carboxymethyl-β-D-glucopyranoside and 2,3,4,6-tetra-O-acetyl-1-carboxypropyl-β-D-glucopyranoside.

2. Compound according to claim 1, in which R₁ is an alkyl radical or acyl radical and/or R₂ is an alkyl radical or acyl radical.

3. Compound according to claim 1 or 2 **characterised in that** the carbohydrate (KH) is glucose, fructose, saccharose, maltose, palatinose, raffinose, lactose, trehalulose, a polyfructane or a polyglucane.

4. Compound according to claim 3 in which the polyfructane is inulin.

5. Compound according to claim 3 in which the polyglucane is cellulose, starch or maltodextrin.

6. Compound according to one of claims 1 to 5 **characterised in that** if the carbohydrate (KH) is a monosaccharide, n = 1, if KH is a disaccharide or trisaccharide, n = 1 to 3 and if KH is a polysaccharide, n = 0.2 to 1.

7. Compound according to one of claims 1 to 6 **characterised in that** the carbohydrate (KH) is fully acylated.

8. Compound according to claim 7 **characterised in that** the carbohydrate (KH) is fully acetylated.

9. Compound according to one of claims 1 to 8 **characterised in that** R₁ is equal H with p = 0 or p = 1.

10. Process for the production of acetylated carbohydrates having at least one etherified carboxyalkyl group according to one of claims 1 to 9, in which carboxyalkylated carbohydrates are reacted with carboxylic anhydrides or carboxylic acid chlorides in the presence of a catalyst.

11. Process according to claim 10 **characterised in that** the carbohydrate (KH) is a monosaccharide, disaccharide, trisaccharide or polysaccharide, the carboxyalkyl group is a carboxymethyl or carboxyethyl group and in which carboxylic acid anhydrides or carboxylic acid chlorides of C2 to C10 are used.

12. Process according to claim 11 **characterised in that** carboxylic acid anhydrides or carboxylic acid chlorides of C2 to C6 carboxylic acids are used.

13. Process according to one of claims 10 to 11 **characterised in that** the catalyst is pyridine, 4-dimethylaminopyridine, sodium acetate, zinc chloride or a Broenstedt acid.

14. Use of acylated carbohydrates having at least one etherified carboxyalkyl group according to one of claims 1 to 9 in washing agents as complexing agents with a simultaneous bleaching agent-activating effect.

## Revendications

1. Hydrates de carbone acylés comportant au moins un groupe carboxyalkyle éthérifié avec l'hydrate de carbone, de formule générale
KH [-O-CHR₁-(CH₂)ₚ - (COOH ou COO⁻)]ₙ [-O-CO-R₂]ₘ
dans laquelle KH (hydrate de carbone) est un monosaccharide, un disaccharide, un trisaccharide ou un polysaccharide, et
lorsque KH est un monosaccharide, n = 1 à 4 et m = 1 à 4, avec n + m = 2 à 5,
lorsque KH est un disaccharide, n = 1 à 7 et m = 1 à 7, avec n + m = 2 à 8,
lorsque KH est un trisaccharide, n = 1 à 10 et m = 1 à 10, avec n + m = 2 à 11, et
lorsque KH est un polysaccharide, n = 0,2 à 2,8 et m = 0,2 à 2,8 avec n + m = 0,4 à 3 (polysaccharide sur la base du motif monosaccharide du KH), et
dans laquelle R₁ = H ou R₁ est un radical comportant 1 à 9 atomes de carbone, et
R₂ est un radical comportant 1 à 9 atomes de carbone et p = 0 à 9, à l'exception du 2,3,6,2',3',4',6'-heptaacétyl-1-carboxyméthyl-β-lactose, du 2,3,6,2',3',4',6'-heptaacétyl-1-carboxypropyl-β-lactose, du 2,3,4,6-tétra-O-acétyl-1-carboxyméthyl-β-D-glucopyranoside et du 2,3,4,6-tétra-O-acétyl-1-carboxypropyl-β-D-glucopyranoside.

2. Composé selon la revendication 1, dans lequel R₁ est un radical alkyle ou acyle et/ou R₂ est un radical alkyle ou acyle.

3. Composé selon la revendication 1 ou 2, **caractérisé en ce que** l'hydrate de carbone (KH) est un glucose, un fructose, un saccharose, un maltose, un palatinose, un raffinose, un lactose, un tréhalulose, un polyfructane ou un polyglucane.

4. Composé selon la revendication 3, dans lequel le polyfructane est l'inuline.

5. Composé selon la revendication 3, dans lequel le polyglucane est la cellulose, l'amidon ou une maltodextrine.

6. Composé selon l'une des revendications 1 à 5, **caractérisé en ce que** lorsque l'hydrate de carbone (KH) est un monosaccharide, n = 1, lorsque KH est un disaccharide ou un trisaccharide, n = 1 à 3 et lorsque KH est un polysaccharide, n = 0,2 à 1.

7. Composé selon l'une des revendications 1 à 6, **caractérisé en ce que** l'hydrate de carbone (KH) est entièrement acylé.

8. Composé selon la revendication 7, **caractérisé en ce que** l'hydrate de carbone (KH) est entièrement acétylé.

9. Composé selon l'une des revendications 1 à 8, **caractérisé en ce que** R₁ vaut H, avec p = 0 ou p = 1.

10. Procédé de préparation d'hydrates de carbones acylés, comportant au moins un groupe carboxyalkyle éthérifié, selon l'une des revendications 1 à 9, dans lequel les hydrates de carbone carboxyalkylés sont mis à réagir avec des anhydrides d'acides carboxyliques ou des chlorures d'acides carboxyliques en présence d'un catalyseur.

11. Procédé selon la revendication 10, **caractérisé en ce que** l'hydrate de carbone (KH) est un mono-, un di-, un trisaccharide ou un polysaccharide, le groupe carboxyalkyle est un groupe carboxyméthyle ou carboxyéthyle, et dans lequel on utilise des anhydrides d'acides carboxyliques ou des chlorures d'acides carboxyliques en C2 à C10.

12. Procédé selon la revendication 11, **caractérisé en ce que** l'on utilise des anhydrides d'acides carboxyliques ou des chlorures d'acides carboxyliques en C2 à C6.

13. Procédé selon l'une des revendications 10 à 11, **caractérisé en ce que** le catalyseur est de la pyridine, de la 4-diméthylaminopyridine, de l'acétate de sodium, du chlorure de zinc ou un acide de Brönstedt.

14. Utilisation d'hydrates de carbones acylés, présentant au moins un groupe carboxyalkyle éthérifié, selon l'une des revendications 1 à 9 dans des détergents, en tant qu'agents de complexation présentant simultanément un effet d'activation de l'agent de blanchiment.
